# EUROPEAN PATENT SPECIFICATION

(11) **EP 0 723 789 B1**
(45) Date of publication and mention of the grant of the patent: **14.05.2003**
(21) Application number: 95119952.0
(22) Date of filing: 18.12.1995
(51) Int. Cl.: A61N 1/375

(54) **Locking device**
Verschlussverbindung
Fermeture

(30) Priority: 26.01.1995 SE 9500273
(43) Date of publication of application: 31.07.1996
(73) Proprietor: St. Jude Medical AB, 175 84 Järfälla (SE)
(72) Inventor: Fröberg, Paul, S-161 70 Bromma (SE); Dahlberg, Kenneth, S-112 26 Stockholm (SE)

(56) References cited:
- EP-A- 0 448 760
- US-A- 3 295 872
- US-A- 4 027 678
- US-A- 5 086 773

## Description

The present invention relates to a locking device with a helix coil which can assume a first, locking position, in which it grips a rod or pin inserted into the coil to prevent longitudinal movement of the rod or pin, and a second, release position in which said rod or pin is free to move in and out of the coil, the first position being assumed when the coil is not influenced in its helix widening direction and the second position being assumed when the coil is influenced in its helix direction by rotation in its unwinding direction The general object of the invention is to set forth a solution to the problem of securing e.g. a pin for a device without the use of a tool, such as a screwdriver for securing the pin with a special screw.

According to one special application, the invention sets forth a locking device for locking and establishing contact for an electric conductor's contact pin. This electrical conductor could be an electrode lead for a medical implant, e.g. a pacemaker or defibrillator.

A pacemaker's connecting parts often consist of a connector housing with attendant electrode connections, the connector housing being made of a transparent epoxy plastic affixed to the top of the pacemaker capsule. The electrode connections in the connector housing are electrically connected to the pacemaker circuits in the capsule via a plurality of connecting pins. The proximal end of the electrode lead has a contact pin which is secured inside the connector housing with e.g. screws. The American patent document US-5 086 773 provides examples of such solutions in its description of the prior art. The device subsequently described in US-5 086 773 is an example of the way the contact pin can be secured without any screws or tools. The locking device described there contains at least one helix coil arranged in a connection hole for locking the contact pin. The internal diameter of the coil is somewhat smaller than the external diameter of the contact pin. When the contact pin is rotated part of one revolution in the coil's unwinding direction at the same time as the contact pin is pressed into the connection hole, the coil expands enough to admit the contact pin. When the contact pin is released, the coil strives to resume its normal position, with the result that the coil grips the contact pin, thereby locking the electrode lead's contact pin and establishing electrical contact.

EP-0 448 760 also provides examples of a locking device in which a coil is installed in a connection hole, in the same way as in US-5 086 773, to lock and establish electrical contact for a contact pin on an electrical conductor in the connection hole.

The innermost end section of the locking coil used in these known devices is mounted in some way in the connection hole. One problem with these devices is that if the contact pin, when locked by the locking device, is inadvertently rotated in the coil's winding direction, i.e. opposite its unwinding direction, the coil might break.

The object of the present invention is to set forth a device which solves this problem.

This object is achieved with a locking device of the above-described kind and with the features set forth in the second part of the first patent claim.

Thus, the invention achieves a locking device with a blocking means which only prevents the coil from rotating in its unwinding direction around its longitudinal axis in relation to the locking device, thereby eliminating the problem of broken springs with the known devices.

The invention will now be described in greater detail, referring to the FIGURES in the attached drawings:
FIG. 1 is a schematic view of a special application of a locking device;
FIG. 2 is the helix coil according to the invention;
FIG. 3 shows two embodiments of blocking means according to the invention;
FIG. 4 shows the contact pin affixed by the locking device according to the invention.

The same reference designations are used for the same items in the FIGURES.

To facilitate understanding of the invention, it will now be described in greater detail in conjunction with a special application in which the locking device is used to lock an electrical conductor's contact pin.

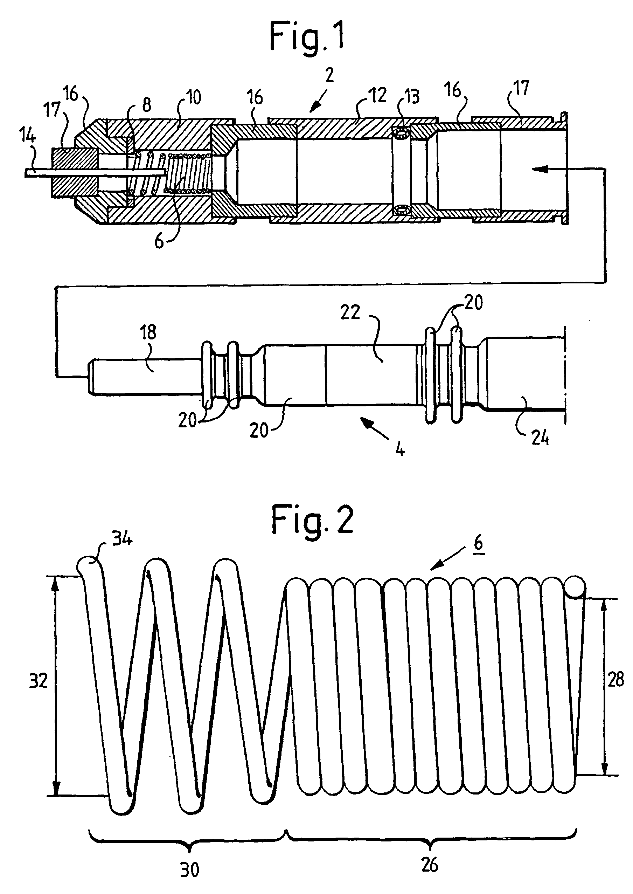

FIG. 1 is a schematic rendition of a locking device 2 according to the invention and an electrical conductor 4 which is to be locked by the locking device 2. The locking device 2 comprises a helix coil 6 and a blocking means 8. The locking device 2 further comprises a first contact 10, a second contact 12 with a contact coil 13, a third contact 14, insulating parts 16 and support parts 17. The electrical conductor 4 comprises a contact pin 18, insulated areas with flanges 20, a connection 22 and an insulated cable 24. The flanges are made of a resilient material which help secure the electrical conductor 4 in the locking device 2.

FIG. 2 shows the helix coil 6. It has a fixation section 26 in which the coil is tightly coiled in 10-15 turns, preferably 12 turns, with no spacing between turns. The fixation section 26 has a first internal diameter 28 which is slightly smaller than the diameter of the contact pin 18. After 10-15 turns, the fixation section 26 changes into a pressure section 30 in which the coil 6 is loosely coiled with a greater pitch than in the fixation section 26. The pressure section 30 has fewer turns, i.e. 2-5 turns, preferably 3 turns, than the fixation section 26. The pressure section 30 has a second internal diameter 32 which is slightly larger than the diameter of the contact pin 18. The pressure section 30 terminates with an end section 34.

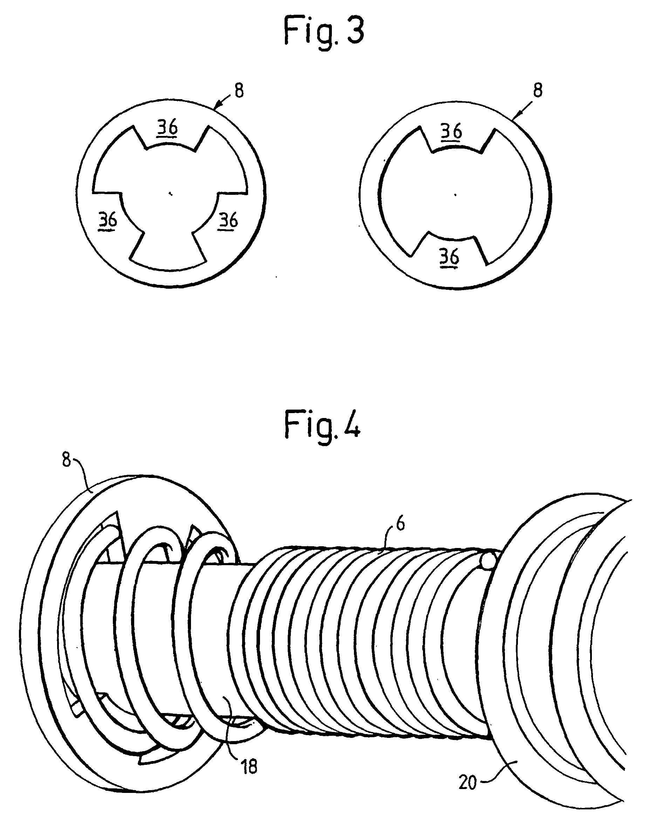

FIG. 3 shows two embodiments of the blocking means 8. The blocking means 8 consists of a washer with a hole in the middle. The diameter of the hole is slightly larger than the external diameter of the pressure section 30. A plurality of blocking parts 36 are arranged in the center of the washer. The FIGURE shows blocking means 8 with two and three blocking parts respectively 36, but only one blocking part 36 is or more than three blocking parts 36 are possible, of course. The blocking parts 36 can be described as tabs whose innermost edges, if connected by imaginary arcs, would form a circle with a diameter less than the internal diameter 32 of the pressure section 30 but larger than the diameter 18 of the contact pin.

FIG. 4 shows the contact pin 18 locked in place with the locking device according to the invention.

Referring to the FIGURES, the invention's function will now be described in greater detail.

The coil spring 6 is constrained under tension inside a space capped by the blocking means 8 at one end and the insulating part 16 nearest the coil 6 at the other end. The blocking means 8 is mounted by the combination of a weld and clamp so only blocking parts 36 are inside the inner walls of the locking device 2. The coil 6 is arranged so the pressure section 30 is slightly compressed and so the coil 6, the end section 34 in particular, presses against the blocking parts 36.

When the electrical conductor 16 with the contact pin 18 is to be secured by the locking device 2, it is pressed into the spring 6 at the same time as it is rotated in the unwinding direction, causing the coil 6 to expand in its helix widening direction and assume a release position in relation to its longitudinal axis. The helix widening direction refers to a radial direction out from the coil's longitudinal axis. So the contact pin 18 is inserted into the coil 6 while simultaneously being rotated. Rotating the contact pin 18 less than one revolution is enough to cause expansion of the coil 6. The coil's end section 34 presses against the blocking means's 8 blocking parts 36 when the contact pin 18 is rotated in the unwinding direction, thereby preventing the spring 6 from rotating more than part of one revolution.

When the coil 6 is not influenced in its helix widening direction, i.e. when rotation of the contact pin 18 in the unwinding direction ceases and the contact pin has been introduced into the coil 6, the coil 6 assumes a locking position in relation to its longitudinal axis, thereby locking the contact pin 18 in place.

When the electrical conductor 4 with the contact pin 18 is to be detached from the locking device 2, the contact pin 18 is rotated in the coil's 6 unwinding direction, i.e. the same rotation direction as in locking, thereby influencing the coil 6 in its helix widening direction. The coil 6 will then return to its release position in relation to its longitudinal axis and relax its grip on the contact pin 18 which can then be withdrawn.

If the coil 6 with the locked contact pin 18 is rotated in the coil's 6 winding direction, the coil 6 will rotate with the contact pin 18, since the end section 34 will slide over the blocking parts 36 and fail to prevent rotation. This accordingly eliminates the problem of coils broken by twisting, as found in locking devices with a permanently mounted coil.

Establishing electrical contact with the electrical conductor 4 is performed by a connection to the first contact 10 to which the blocking means 8 is electrically connected. Since the spring 6 is under tension, it constantly presses against the blocking means 8 and guarantees contact with the contact pin 18 by the fixation section's 26 grip on the contact pin 18. Electrical contact can also be made with the second contact 12 which, via the contact coil 13, presses against the connection 22 on the conductor 4. The third contact 14 consists of a connection pin, concentrically arranged in the locking device 2, which contacts the contact pin 18 when the connection pin is inserted into an opening (not shown) in the contact pin 18. This third contact 14 can be used to indicate that the contact pin has been correctly locked and electrical contact established.

Within the scope of the invention, the pressure section 30 of the coil 6 can have a conical shape with a first internal diameter 28 in the fixation section 26, said diameter increasing at the transition to the pressure section 30 to a second internal diameter 32 at the end of the pressure section 30.

The terminal part of the pressure section 30 up to the end section 34 can be devised in a plurality of different ways. For example, it can have a defined bend towards the blocking means 8. Another possibility is for the terminal part of the pressure section 30 up to the end section 34 to have a larger diameter than the rest of the pressure section 30 and interact with the blocking means 8, which has blocking parts 36 which can consist of notches or grooves on the inside of the locking device 2.

According to the above, the special application of the invention relates to the locking and electrical contact of an electrical conductor for a medical implant. The implant can consist of a pacemaker or defibrillator. The length of the coil 6 used in this special application is about 3 mm, the first internal diameter 28 being about 1.45 mm and the second internal diameter 32 being about 1.8 mm. The diameter of the contact pin is about 1.6 mm.

According to a second special application of the invention, the locking device is employed to lock and establish electrical contact for a contact pin for an electrical conductor.

According to a third special application of the invention, the locking device is only used for mechanically locking the pin of an apparatus or some other device. Application of the invention would be much easier when locking is the only function of interest, since no consideration need then be paid to establishing electrical contact.

### Reference designations

- 2: Locking device
- 4: Electrical conductor
- 6: Helix coil
- 8: Blocking means
- 10: First contact
- 12: Second contact
- 13: Contact coil
- 14: Third contact
- 16: Insulating section
- 17: Supporting parts
- 18: Contact pin
- 20: Insulating part with flanges
- 22: Connection
- 24: Insulated cable
- 26: Fixation section
- 28: First internal diameter
- 30: Pressure section
- 32: Second internal diameter
- 34: End section
- 36: Blocking part

## Claims

1. A locking device (2) with a helix coil (6) which can assume a first, locking position, in which it grips a rod or pin inserted into the coil to prevent longitudinal movement of the rod or pin, and a second, release position in which the said rod or pin is free to move in and out of the coil, the first position being assumed when the coil (6) is not influenced in its helix widening direction and the second position being assumed when the coil (6) is influenced in its helix widening direction by rotation in its unwinding direction,**characterized in that** the locking device (2) has a blocking means (8) which only prevents the coil (6) from rotating in its unwinding direction around its longitudinal axis in relation to the locking device (2).

2. A locking device (2) according to claim 1, characterized i n that the helix coil (6) has a tightly coiled fixation section (26) and a loosely coiled pressure section (30) which is shorter than the fixation section (26), and the pressure section (30) terminates with an end section (34).

3. A locking device (2) according to claim 2, characterized i n that the fixation section (26) has a smaller internal diameter than the pressure section (30).

4. A locking device (2) according to claim 2 or 3, **characterized in that** the blocking means (8) has at least one blocking part (36) which interacts with the end section (34) to prevent the coil (6) from rotating in its unwinding direction around the coil's longitudinal axis in relation to the locking device (2).

5. A locking device (2) according to claim 2, **characterized in that** the pressure section (30) has a conical shape with a first internal diameter (28) in the fixation section (26), said diameter increasing at the transition to the pressure section (30) to a second internal diameter (32) at the end of the pressure section (30).

6. A locking device (2) according to any of claims 2 to 5, **characterized in that** the terminal part of the pressure section (30) up to the end section (34) has a defined bend towards the blocking means (8).

7. A locking device (2) according to any of claims 4 to 6, **characterized in that** the terminal part of the pressure section (30) up to the end section (34) has a larger diameter than the rest of the pressure section (30), the end section (34) interacting with the blocking means' (8) blocking parts (36) which consist of notches or grooves on the inside of the locking device (2).

8. A locking device (2) according to any of claims 1 to 7, **characterized in that** the locking device (2) is arranged to lock an electrical conductor's contact pin.

9. A locking device (2) according to any of claims 1 to 7, **characterized in that** a locking device (2) is arranged to lock the contact pin of an apparatus.

10. A locking device (2) according to claim 9, **characterized in that** the apparatus is a medical implant, e.g. a pacemaker, and the locking device (2) is arranged to lock the contact pin of an electrode lead for the medical implant.

## Patentansprüche

1. Verriegelungsvorrichtung (2) mit einer schraubenlinienförmigen Wicklung (6), die eine erste, verriegelnde Position einnehmen kann, in der sie einen in die Wicklung eingesetzten Stab oder Stift festhält, um eine Längsbewegung des Stabes bzw. des Stiftes zu verhindern, und eine zweite, freigebende Position, in der der genannte Stab oder Stift freigegeben ist, um sich in die oder aus der Wicklung zu bewegen, wobei die erste Position angenommen wird, wenn die Wicklung (6) in einer ihre Schraubenlinie erweiternden Richtung nicht beeinflußt wird und die zweite Position angenommen wird, wenn die Wicklung (6) in einer ihre Schraubenlinie erweiternden Richtung durch Verdrehen in ihre Abwickelrichtung beeinflußt wird, **dadurch gekennzeichnet , dass** die Verriegelungsvorrichtung (2) ein Blockiermittel (8) aufweist, das die Wicklung (6) nur daran hindert sich um ihre Längsachse gegenüber der Verriegelungsvorrichtung (2) in ihrer Abwickelrichtung zu drehen.

2. Verriegelungsvorrichtung (2) nach Anspruch 1, **dadurch gekennzeichnet , dass** die schraubenlinienförmige Wicklung (6) einen dicht gewickelten Festlegungsabschnitt (26) und einen lose gewickelten Druckabschnitt (30) aufweist, der kürzer als der Festlegungsabschnitt (26) ist und dass der Druckabschnitt (30) in einem Endabschnitt (34) endet.

3. Verriegelungsvorrichtung (2) nach Anspruch 2, **dadurch gekennzeichnet , dass** der Festlegungsabschnitt (26) einen kleineren Innendurchmesser als der Druckabschnitt (30) aufweist.

4. Verriegelungsvorrichtung (2) nach Anspruch 2 oder 3, **dadurch gekennzeichnet , dass** das Blockiermittel (8) wenigstens einen Blockierteil (36) aufweist, der mit dem Endabschnitt (34) zusammenwirkt, um die Wicklung (6) daran zu hindern, sich gegenüber der Verriegelungsvorrichtung (2) in ihre Abwickelrichtung um die Längsachse der Spule zu drehen.

5. Verriegelungsvorrichtung (2) nach Anspruch 2, **dadurch gekennzeichnet , dass** der Druckabschnitt (30) eine konische Form mit einem ersten Innendurchmesser (28) im Festlegungsabschnitt (26) aufweist und dieser Durchmesser am Übergang zum Druckabschnitt (30) bis zu einem zweiten Innendurchmesser (32) am Ende des Druckabschnittes (30) zunimmt.

6. Verriegelungsvorrichtung (2) nach einem der Ansprüche 2 bis 5, **dadurch gekennzeichnet , dass** der Endteil des Druckabschnittes (30) bis zum Endabschnitt (34) eine definierte Biegung zum Blockiermittels (8) hinaufweist.

7. Verriegelungsvorrichtung (2) nach einem der Ansprüche 4 bis 6, **dadurch gekennzeichnet , dass** der Endteil des Druckabschnittes (30) bis zum Endabschnitt (34) einen größeren Durchmesser als der Rest des Druckabschnittes (30) aufweist, wobei der Endabschnitt (34) mit den Blockierteilen (36) des Blockiermittels (8) zusammenwirkt, welche aus Kerben oder Nuten an der Innenseite der Verriegelungsvorrichtung (2) gebildet sind.

8. Verriegelungsvorrichtung (2) nach einem der Ansprüche 1 bis 7, **dadurch gekennzeichnet , dass** die Verriegelungsvorrichtung (2) ausgebildet ist, um den Kontaktstift eines elektrischen Leiters festzuhalten.

9. Verriegelungsvorrichtung (2) nach einem der Ansprüche 1 bis 7, **dadurch gekennzeichnet , dass** die Verriegelungsvorrichtung (2) ausgebildet ist, um den Kontaktstift eines Geräts festzuhalten.

10. Verriegelungsvorrichtung (2) nach Anspruch 9, **dadurch gekennzeichnet , dass** das Gerät ein medizinisches Implantat, beispielsweise ein Schrittmacher ist, und die Verriegelungsvorrichtung (2) ausgebildet ist, um den Kontaktstift einer Elektrodenleitung für das medizinische Implantat festzuhalten.

## Revendications

1. Dispositif de blocage (2) ayant une bobine hélicoïdale (6) qui peut adopter une première position de blocage, dans laquelle il serre une tige ou broche introduite dans la bobine pour empêcher un mouvement longitudinal de la tige ou broche, et une deuxième position de relâchement dans laquelle la tige ou broche est libre de se déplacer à l'intérieur et à l'extérieur de la bobine, la première position étant adoptée lorsque la bobine (6) n'est pas influencée dans son sens d'élargissement hélicoïdal et la deuxième position étant adoptée lorsque la bobine (6) est influencée dans son sens d'élargissement hélicoïdal par rotation dans son sens de déroulement, **caractérisé en ce que** le dispositif de blocage (2) comporte un moyen de blocage (8) qui empêche seulement la bobine (6) de tourner dans son sens de déroulement autour de son axe longitudinal par rapport au dispositif de blocage (2).

2. Dispositif de blocage (2) suivant la revendication 1, **caractérisé en ce que** la bobine hélicoïdale (6) comporte une partie de fixation (26) étroitement enroulée et une partie de pression (30) enroulée de façon lâche qui est plus courte que la partie de fixation (26) et **en ce que** la partie de pression (30) se termine par une partie d'extrémité (34).

3. Dispositif de blocage (2) suivant la revendication 2, **caractérisé en ce que** la partie de fixation (26) présente un diamètre intérieur inférieur à celui de la partie de pression (30).

4. Dispositif de blocage (2) suivant la revendication 2 ou 3, **caractérisé en ce que** le moyen de blocage (8) comporte au moins une pièce de blocage (36) qui interagit avec la partie d'extrémité (34) afin d'empêcher la bobine (6) de tourner dans son sens de déroulement autour de l'axe longitudinal de la bobine par rapport au dispositif de blocage (2).

5. Dispositif de blocage (2) suivant la revendication 2, **caractérisé en ce que** la partie de pression (30) présente une forme conique avec un premier diamètre intérieur (28) dans la partie de fixation (26), ce diamètre s'accroissant au niveau de la transition vers la partie de pression (30) en un deuxième diamètre intérieur (32) à l'extrémité de la partie de pression (30).

6. Dispositif de blocage (2) suivant l'une quelconque des revendications 2 à 5, **caractérisé en ce que** la partie terminale de la partie de pression (30) jusqu'à la partie d'extrémité (34) présente une courbure définie vers le moyen de blocage (8).

7. Dispositif de blocage (2) suivant l'une quelconque des revendications 4 à 6, **caractérisé en ce que** la partie terminale de la partie de pression (30) jusqu'à la partie d'extrémité (34) présente un diamètre supérieur au reste de la partie de pression (30), la partie d'extrémité (34) interagissant avec les pièces de blocage (36) du moyen de blocage (8) qui se composent d'encoches ou de rainures à l'intérieur du dispositif de blocage (2).

8. Dispositif de blocage (2) suivant l'une quelconque des revendications 1 à 7, **caractérisé en ce que** le dispositif de blocage (2) est disposé pour bloquer une broche de contact de conducteur électrique.

9. Dispositif de blocage (2) suivant l'une quelconque des revendications 1 à 7, **caractérisé en ce qu'**un dispositif de blocage (2) est disposé pour bloquer la broche de contact d'un appareil.

10. Dispositif de blocage (2) suivant la revendication 9, **caractérisé en ce que** l'appareil est un implant médical, par exemple un stimulateur cardiaque, et **en ce que** le dispositif de blocage (2) est disposé pour bloquer la broche de contact d'un conducteur d'électrode destinée à l'implant médical.
